# EUROPEAN PATENT APPLICATION

(11) **EP 2 105 144 A1**
(43) Date of publication of application: **30.09.2009**
(21) Application number: 07828023.7
(22) Date of filing: 16.11.2007
(51) Int. Cl.: A61K 47/36, A23L 1/00, A61K 9/36, A61K 9/62, A61K 47/10, A61K 47/12, A61K 47/14

(54) **CHITOSAN SOLUTION AND MEDICAL PREPARATION WITH CHITOSAN COATING FORMED FROM THE SOLUTION**

(30) Priority: 21.12.2006 JP 2006343728
(71) Applicant: Aicello Chemical Co., Ltd., Toyohashi-shi Aichi 441-1115 (JP); Freund Corporation, Tokyo 163-6034 (JP)
(72) Inventor: ITO, Katsuhito, Toyohashi-shi Aichi 441-1115 (JP); MIZOBATA, Kazuyuki, Toyohashi-shi Aichi 441-1115 (JP); KAMIMURA, Motokazu, Toyohashi-shi Aichi 441-1115 (JP); SHOBU, Tomoyuki, Hamamatsu-shi Shizuoka 431-2103 (JP)
(74) Representative: HOEIBERG A/S
(86) International application number: PCT/JP2007/001246
(87) International publication number: WO 2008/075448

(57) **Abstract**

A chitosan coating solution which is so safe that it is capable of oral administration in the fields of foods and medicines; a chitosan coating film reduced in the content of a residual organic acid and having satisfactory water resistance; and a coated medical preparation having the coating film. The coating solution is **characterized by** being obtained by incorporating a glycerin fatty acid ester into an aqueous chitosan solution prepared through dissolution with a volatile organic acid. The chitosan coating film is one obtained by drying and solidifying the solution through film coating. The organic acid used in the dissolution remains in the coating film only in a small amount and the film has satisfactory water resistance. When the chitosan coating solution, which contains a glycerin fatty acid ester and is a highly safe solution prepared through dissolution with a volatile organic acid, is subjected to film coating, a film is formed and, simultaneously therewith, the organic acid continuously volatilizes. Consequently, a chitosan coating film having water resistance can be efficiently obtained. Furthermore, by forming a known enteric coating film in combination with the coating film on the outer side thereof, an application to large-intestine drug delivery system preparations can be expected with ease.

## Description

### Technical Field

The present invention relates to a highly safe chitosan coating solution for orally administrable preparations for the fields of food and pharmaceutical products, and also to a practically useful chitosan coating film obtainable by a process of film coating, which has a reduced amount of residual organic acid and satisfactory resistance to water and Japanese Pharmacopoeia second fluid, and a coated preparation containing the chitosan coating film.

### Background Art

Chitosan, which is a naturally occurring polymer, and chitin, which is the raw material of chitosan, have high degradability by microorganisms, biocompatibility, biodegradability, and properties such as absorbability and selective permeability, which properties are not exhibited by general synthetic polymers. Therefore, attention has been paid to these materials as functional polymer materials, and research is being actively conducted on the applications thereof in the fields of food and pharmaceutical products, as biodegradable films, medical materials, separation membranes and the like.

Chitosan is a straight-chained alkaline polysaccharide in which 2-amino-2-deoxy-D-glucose is linked via β-1,4-glucoside bond, and exists in the cell walls of filamentous fungi in nature. From the industrial standpoint, chitosan can be obtained by treating the β-1,4-polymer of 2-acetamido-2-deoxy-D-glucose of natural chitin, which is contained in large quantities in the shells of crabs and shrimps, with concentrated alkali, and subjecting the resultant to complete or partial deacetylation.

Since chitosan is not thermoplastic, chitosan is first dissolved in an aqueous solution of acetic acid, dilute hydrochloric acid or the like, and then, formed products of chitosan, such as films, fibers or capsules, can be obtained. These formed products of chitosan are frequently utilized as the polymer materials for formation of biomaterials, bases for drug delivery, separation membranes, edible films or the like. Particularly, when a chitosan-based formed product is used as a large intestine drug delivery system device in combination with an enteric base, the chitosan-based formed product is required to have sufficient, water resistance in the interior of the small intestine after the enteric base has been dissolved in the small intestine environment. Furthermore, in the fields of food and pharmaceutical products, it is necessary to secure sufficient safety of the raw materials that are used for oral administration.

In the case of taking orally in the fields of food and pharmaceutical products, dosage forms such as tablets, hard capsules, soft capsules, powders and granules are generally used. As for the method of forming coatings on the surface of these various dosage forms, a method called film coating is generally used. However, in the case where a chitosan coating film is obtained by performing film coating with a chitosan solution which has been prepared by dissolving in an aqueous solution of an organic acid, the acid contained in the chitosan solution, although partly evaporates during the drying process, cannot be completely removed, and the acid inevitably remains behind in the coating film in large quantities. As a result, the water resistance of the chitosan coating film is lowered, and there is a problem that the residual acid further promotes the proceeding of hydrolysis over time or acetylation of chitosan, and causes coloration or deterioration of properties of the formed products under long-term storage. In addition, it is also feared that the acid remaining in the chitosan coating film may affect the preparations such as tablets, hard capsules or soft capsules themselves, or the contents thereof.

Patent Document 1 describes, in order to address such problems, a post-treatment of immersing the formed products in an aqueous alkali solution such as of sodium hydroxide to neutralize the residual acid, and removing the excess alkali and generated salts by washing with water. However, such a post-treatment generates another necessity to treat the alkali remaining in the products, and it is difficult to use this technique, particularly in the applications associated with food and pharmaceutical products, where remaining of alkali is often not allowed.

To address such problems, Patent Document 2 describes a method of immersing the formed products in a water-miscible organic solvent, and extracting the residual acid for removal. However, because products coated with a chitosan coating film are immersed in a water-miscible organic solvent, the solvent may unpreferably have adverse effects on the contents.

On the other hand, Patent Document 3 describes a method of reducing the content of residual acid by adding triethylene glycol to an organic acid aqueous solution of chitosan, and drying and solidifying the solution so that the added triethylene glycol accelerates evaporation of acid during the drying process. However, since the additive that can be used is limited to triethylene glycol, the possibility of use thereof in the fields of food and pharmaceutical products is limited. Furthermore, the temperature at which the chitosan-based formed products are heated and dried to remove acid is as high as 100°C or above, and there is concern for the adverse effects on the contents and the requirement of large energy consumption.

Patent Document 4 describes a method of obtaining a water-resistant chitosan coating film by suspending chitosan and partially deacetylated chitin in water, dissolving the suspension by aeration with carbon dioxide gas, and then drying and solidifying the solution. However, since the shape of the chitosan or partially deacetylated chitin is limited to a powdered form, workability is deteriorated. Furthermore, hydrochloride salts is formed during preparing a chitosan gel, and thus salts are suspected to remain in the resulting chitosan coating film. Therefore, when it is envisaged to use the method in the fields of food and pharmaceutical products, it is not preferable from the aspect of safety.

Patent Document 5 describes a water-soluble film coated preparation for food, which utilizes organic acid salts or hydrochloride of the polymer material obtainable by deacetylation of chitin, and which is aimed for preservation of food, moisture retention and masking of taste. However, because the organic acid salts or hydrochloride thus formed remains in the chitosan coating film, water resistance is not sufficient, and there is a possibility of having a problem in the stability over time, due to the remaining of acid.

Patent Document 6 reports a structure which enables delivery of active ingredients to the large intestine, and there is described a method of obtaining a water-resistant chitosan coating film by adding glycerin, propylene glycol, sugar esters or the like as additives to chitosan solutions. However, when these additives are used, since the coating film has high flexibility due to the plasticizing effect, and also, the additives themselves are extremely hydrophilic, the strength of the coating film is weakened. In addition, large amounts of acid also remain behind, and therefore, the water resistance of the chitosan is not sufficient.
[Patent Document 1] JP-B-S52-41797
[Patent Document 2] JP-A-H06-25433
[Patent Document 3] JP-A-H07-179623
[Patent Document 4] JP-A-2002-241405
[Patent Document 5] JP-A-H06-319508
[Patent Document 6] JP-A-H10-324642

### Disclosure of the Invention

### Problems to be Solved by the Invention

The present invention has been achieved under such circumstances, and an object of the invention is to provide a highly safe chitosan coating solution containing additives which can be used in the fields of food and also pharmaceutical products, and another object is to provide a chitosan coating film with excellent water resistance formed by film coating of the solution, which coating has a reduced amount of residual organic acid which has been used in the dissolution of chitosan, does not particularly require a post-treatment for removing organic acids, and has excellent production efficiency.

### Means for Solving the Problems

The inventors of the present invention devotedly conducted investigations to achieve the above-described objects. As a result, it has been found that a chitosan coating film obtainable by drying and solidifying a chitosan coating solution through the process of film coating, the chitosan coating solution being **characterized in that** 5% by weight to 200% by weight of a glycerin fatty acid ester with respect to the weight of chitosan is added to an aqueous solution of chitosan dissolved with the use of a volatile organic acid, has an extremely low content of acid, and has sufficient water resistance, so that the chitosan coating film can be used even in the fields of food and pharmaceutical products. Thus, the inventors completed the present invention.

### Effects of the Invention

According to the present invention, a volatile organic acid aqueous solution of chitosan is combined with a glycerin fatty acid ester, or foam is further incorporated, therefore, a chitosan coating film having the amount of volatile organic acids remaining in the chitosan coating film efficiently reduced, and maintaining sufficient water resistance, can be obtained. The glycerin fatty acid ester of an additive in the chitosan coating film has fatty acid which has a hydrophobic group, and thus is capable of imparting water resistance, together with a plasticizing effect. As a result, reduction of the amount of use of polyhydric alcohols, which are plasticizers causing a decrease in the film strength, and reduction of the thickness of the coating film, can be realized. Accordingly, the chitosan coating film containing a glycerin fatty acid ester can be expected to exhibit sufficient resistance in the small intestine, and to be applied to large intestine drug delivery system preparations. Furthermore, this thickness reduction of coating film implies shortening of time consumed in the coating process and attainment of high efficiency, and significance thereof is enormous even from the viewpoint of industrialization.

When the technique of the present invention is used, a highly safe chitosan coating film which can be used even in the fields of food and pharmaceutical products, and is less limited in possible uses, can be obtained. Since the chitosan coating film has water resistance, the coating film does not dissolve until it reaches the stomach upon oral administration, and the coating film hardly adheres to the esophageal mucous membrane or the like when swallowing the preparation. Also, in regard to aged people and infants, as well as patients having a problem in swallowing, there can be expected enhancement of safety upon intake of preparations.

### Best Mode for Carrying Out the Invention

The chitosan coating film of the present invention includes all coatings, regardless of the amount of chitosan contained in the coating film, even including those coatings which contain chitosan in the slightest amount. That is, coatings of simple chitosan, as well as composites of chitosan and fillers also fall within the scope of the chitosan coating film. As for the fillers which can be compounded with chitosan, there may be exemplified various starch granules such as corn starch, potato starch and rice starch, powdered or microfibrous celluloses, calcium carbonate, talc, silica and the like. Furthermore a plasticizer may also be added to the chitosan coating film for the purpose of imparting plasticity, and as the plasticizer, any compound can be used depending on the purpose. For example, polyhydric alcohols such as glycerin, diglycerin, diethylene glycol, propylene glycol, triethylene glycol and polyethylene glycol, and the like may be given. Among them, from the viewpoint of uses in the fields of food and pharmaceutical products, it is preferable to use glycerin, propylene glycol and polyethylene glycol.

There is no particular limit in the degree of deacetylation or molecular weight of the chitosan to be used, and any product can be used depending on the purpose. However, in general, upon considering the solubility in acids or the adaptability to coating techniques, products having a degree of deacetylation of 70% by mole or higher are preferred.

The volatile organic acid which assists in water solubilization of chitosan, is required to have sufficient volatility at normal pressure, and as such volatile organic acid, formic acid, acetic acid, propionic acid, trichloroacetic acid and the like may be given. Among them, from the viewpoints of economic efficiency, handlability or safety, acetic acid is most preferable. Chitosan is soluble in aqueous solutions of such organic acids, as well as in aqueous solutions of inorganic acids such as hydrochloric acid, but inorganic acids strongly interact with chitosan and are therefore unsuitable. There is no limit on the amount of the volatile organic acid used, but an amount of 0.8 molar equivalents to 2.0 molar equivalents per amino group of chitosan is suitable. If the amount is less than 0.8 molar equivalents, the solubility of chitosan is low, and if the amount is more than 2.0 molar equivalents, removal of acid cannot be achieved efficiently, and it becomes difficult to obtain a chitosan coating film which exhibits water resistant. Thus, neither case is preferable.

The process of film coating according to the present invention is a method of spraying a coating solution on the surface of a substrate through a spray nozzle or the like, drying and solidifying the solution to form a coating film. The film coating process also includes a method of spray drying a coating liquid containing a substrate, to form a coating film. Examples of the coating apparatus include a coating pan, a through-flow drying type pan coating apparatus, a spout fluidized bed apparatus, a tumbling fluidized bed apparatus, a draft tube spouted bed apparatus, a spray drying granulation apparatus, and the like, and any apparatus that is conventionally used in the relevant technical field may be used. A chitosan coating film can be obtained by drying and solidifying a chitosan solution by using the process of film coating which utilizes these coating apparatuses. The shape of the substrate to be coated is not particularly limited, but may be any shape on which a chitosan coating film can be formed on the solid surface, for example, a tablet, a hard capsule, a soft capsule, a powder or a granule. Furthermore, a chitosan coating in a film form can also be obtained by using a substrate formed from a corrosion resistant material such as stainless steel or plastics, and peeling off the chitosan coating film formed on the surface of the substrate therefrom.

It may be preferable if the chitosan coating solution has a concentration and a viscosity appropriate for the coating apparatus, and preferably, the chitosan concentration is in the range of 0.1% by weight to 20% by weight, while the viscosity is in the range of 10 mPa·s to 1,000 mPa·s (solution temperature 23°C). The coating conditions may be such that the supply air temperature is 40°C to 95°C, while the exhaust air temperature is 30°C to 30°C. If the supply air temperature is lower than 40°C, removal of organic acid is not achieved sufficiently, and thus water resistance cannot be imparted to the chitosan coating film. On the other hand, if the supply air temperature exceeds 100°C, there unpreferably arises a problem in the external appearance, such as coloration or deformation of the chitosan coating film.
Furthermore, a supply air temperature exceeding 100°C in the process of coating an aqueous system, is not common, and is also not practical because the burden loaded on the coating apparatus is increased.

The additive which may be used for efficient removal of the volatile organic acid, essentially comprises glycerin fatty acid esters. The glycerin fatty acid esters include esters of fatty acids and glycerin or polyglycerin, and derivatives thereof. For example, glycerin fatty acid esters, glycerin acetic acid fatty acid esters, glycerin lactic acid fatty acid esters, glycerin citric acid fatty acid esters, glycerin succinic acid fatty acid esters, glycerin acetic acid esters, polyglycerin fatty acid esters, polyglycerin condensed ricinoleic acid esters, and the like can be given. Here, in regard to the fatty acid that is used in the glycerin fatty acid esters, any fatty acid can be used, for example, caprylic acid, capric acid, lauric acid, myristic acid, palmitic acid, stearic acid, behenic acid, oleic acid, linoleic acid, erucic acid, isopalmitic acid, isostearic acid and the like can be given. Among them, fatty acid esters of myristic acid, palmitic acid and stearic acid are preferred. It is preferable if the degree of glycerin polymerization in the polyglycerin fatty acid esters is in the range of monomer to 20-mers, and examples thereof include monoglycerin, diglycerin, triglycerin, tetraglycerin, pentaglycerin, hexaglycerin, decaglycerin, and the like. A monomeric fatty acid has a problem that dissolution in water becomes difficult, while compounds of 21-mer of larger have a problem of rising production costs. Furthermore, as long as the glycerin fatty acid ester can be dissolved or dispersed in water, the degree of substitution of fatty acids is not particularly limited, but the range from monoester to trimester is desirable. It is preferable that the chitosan coating film which is obtained by the process of film coating, contains 5% by weight to 200% by weight of a glycerin fatty acid ester with respect to the weight of chitosan, from the viewpoint of decreasing the amount of residual organic acid. If the amount of addition is less than 5% by weight, the decrease in the amount of residual organic acid is insufficient, and the chitosan coating film becomes less water-resistant. If the addition amount exceeds 200% by weight, the resulting chitosan coating film cannot have sufficient strength, and is not practically useful.

Moreover, foams having a size of about several micrometers in diameter can be stably incorporated into the coating solution by vigorously stirring the chitosan coating solution before or in the middle of the process of film coating, due to the surfactant effect of the glycerin fatty acid ester. In this case, although the volume of the coating solution increases by incorporating foams, if a foaming ratio, which is designated as the value obtained by dividing the volume of this solution containing foams, by the volume of the original solution of before the incorporation of foams, is in the range of greater than 1.0-food to 4.0-fold or less, a further decrease in the amount of residual acetic acid is recognized upon the film coating process, and thus a chitosan coating film having better water resistance and stability over time may be obtained. In addition, a foaming ratio exceeding 4.0-fold is difficult to obtain and is not practical. Since these foams are contained in the mist even during the process of coating, the surface area is increased during drying, and the organic acid is volatilized very efficiently. Accordingly, the amount of residual organic acid in the chitosan coating film which is obtained by drying and solidification through film coating, can be further reduced, as compared to the case where foam is not incorporated. JP-A-H04-144660 describes a method characterized by containing a polyglycerin fatty acid ester and chitosan, emulsifying them with oils and fats, and producing foam, but the present invention is different therefrom because the present invention is characterized by being a uniformly mixed aqueous solution of chitosan, which includes foams formed from glycerin fatty acid ester and chitosan.
As discussed above, since a chitosan coating film comprising 5% by weight to 200% by weight of a glycerin fatty acid ester with respect to the weight of chitosan, efficiently reduces the amount of volatile organic acid in a short time simultaneously with the formation of film through coating, the coating film exhibits water resistance and good external appearance. The chitosan coating film is also applicable to the fields of food and pharmaceutical products, and is highly safe as well as practically useful.

The chitosan coating film of the present invention may also be used in combination with other coating bases, and by forming a coating film of another coating base on the inner layer or the outer layer of the chitosan coating film, a multilayer coated preparation can be obtained. As for the another coating base in the multilayer coating, hypromellose phthalic acid ester, methacrylic acid copolymers, hypromellose, hydroxypropylcellulose, methylcellulose, ethylcellulose, shellac, zein, water-soluble polysaccharides, and oil and fat can be given, but not limited thereto.

Although the chitosan coating film of the present invention disintegrates in the stomach or in the large intestine, the coating film exhibits resistance in the small intestine. Therefore, the coating film can be applied to large intestine drug delivery system preparations by coating an enteric coating base on the outer layer of the chitosan coating film. As for the enteric coating base, hypromellose phthalic acid ester, methacrylic acid copolymers, and aqueous shellac, but not limited thereto.

By combining chitosan with a glycerin fatty acid ester, the chitosan coating film obtained through the process of film coating, has a reduced amount of residual organic acid, and exhibits satisfactory water resistance. The reason for this is not necessarily clear, but because the chitosan coating solution is sprayed into hot air, the surface area is increased, and volatilization of organic acid is accelerated. Furthermore, as the glycerin fatty acid ester or polyhydric alcohol plays a role as a plasticizer, drying of moisture from the chitosan coating film is delayed, and thus volatilization of the volatile organic acid also proceeds simultaneously with the drying of the moisture. If the coating solution contains foams, due to an increase in the surface area, volatilization of the volatile organic acid is further accelerated. Furthermore, since the glycerin fatty acid ester contains fatty acid which is hydrophobic, the chitosan coating film exhibits sufficient water resistance, and thus thickness reduction can be achieved. As discussed above, a chitosan coating film having an extremely lowered amount of residual organic acid and satisfactory water resistance could be obtained by coating a volatile organic acid aqueous solution of chitosan containing a glycerin fatty acid ester.

### Examples

Hereinafter, the present invention will be described more specifically by way of Examples, but the present invention is not intended to be limited to these Examples.

### [Evaluation of samples]

A chitosan coating solution was film coated on uncoated tablets or gelatin hard capsules to prepare a sample, and the external appearance and water resistance of the sample were evaluated, while the amount of organic acid in the chitosan coating film was calculated. An enteric coating was applied to the outer layer of the sample, and the resultants were used as preparation sample to evaluate the function as a large intestine drug delivery system by an in vitro large intestine DDS test. The respective methods for the evaluation of external appearance and water resistance, calculation of the amount of residual organic acid in the chitosan coating film, calculation of the foaming ratio, and the in vitro large intestine DDS test, will be described in the following.

### (Evaluation of external appearance)

The external appearance of the sample was observed visually. The appearance of the sample was judged in four grades for the evaluation of the external appearance.
⊚: The coating film has been well formed over the whole surface and the surface is smooth.
○: The coating film has been well formed, but slight roughness is seen on the surface of the coating film.
Δ: The surface of the coating film is rough.
×: A coating film cannot be formed.

### (Evaluation of water resistance)

The sample was immersed in 60 mL of distilled water for 2 hours and then taken out, and the look of the sample was observed visually. The look of the sample was judged in four grades for the evaluation of water resistance.
⊚: The shape is maintained and the look is well.
○: Slight swelling and deformation is recognized.
Δ: Significant swelling and deformation is recognized, but the contents have been preserved.
×: The contents were released.

### (Calculation of amount of residual organic acid)

The sample was completely dissolved with a sufficient amount of hydrochloric acid, and then an excess amount of sodium hydroxide was introduced. Subsequently, neutralization titration (reverse titration) was further carried out with hydrochloric acid, to thereby determine the number of moles of organic acid in the sample. Then, the value obtained by dividing this number of moles of organic acid by the number of moles of the amino group of chitosan contained in the chitosan coating film of the sample, was designated as the amount of residual organic acid.

### (Calculation of foaming ratio)

In a 500-mL beaker made of glass, 100 g of a coating solution was added, and the coating solution was forcibly stirred by a stirring apparatus to contain foams. The value obtained by dividing the volume of the solution which contained sufficient foams as in this case, by the volume of the original solution which did not contain foams, was designated as the foaming ratio. When the foaming ratio is 1.0-fold, this indicates that the solution does not contain any foam, and as the number increases, it is meant that the solution contains a larger amount of foams.

### (In vitro large intestine DDS test)

The preparation sample was immersed in 100 mL of Japanese Pharmacopoeia first fluid for 2 hours and then taken out, and the look of the preparation sample was observed visually. ○: Both the enteric coating and the chitosan coating film maintain the shape. ×: Both the enteric coating and the chitosan coating disintegrated, and the contents were released. Subsequently, for those samples judged to be ○, each of them was further again immersed in 100 mL of Japanese Pharmacopoeia second fluid for 2 hours, and the look of the preparation sample thereafter was observed visually. ○: The enteric coating disintegrates, and the chitosan coating film maintains the shape. ×: Both the enteric coating and the chitosan coating film disintegrated. Furthermore, each of the samples judged to be ○ was immersed in 100 mL of an acetate buffer solution (Michaelis buffer solution) with pH = 3.5, and it was confirmed that the chitosan coating film disintegrated, and the contents were released. ○: The chitosan coating film disintegrated, and the contents were released. ×: The contents were not released.

### Example 1

A chitosan having a degree of deacetylation of 81 % by mole (manufactured by Katakura Chikkarin Co., Ltd.) was dissolved in water to a concentration of 4% by weight, after adding acetic acid so that the amount reached 1.0 molar equivalent based on the amino group of the chitosan. Then, decaglycerin monopalmitic acid ester (manufactured by Mitsubishi-Kagaku Foods Corp.) was added in an amount of 100% by weight with respect to the weight of chitosan, and the mixture was sufficiently stirred to obtain a chitosan solution. This solution was film coated on uncoated tablets having a diameter of 8 mm by using a film coating apparatus, Hicoater HC-LABO (manufactured by Freund Corporation), and thus samples 1 to 3, each having a coating film whose thickness was such that the weight of the coating film was equivalent to 2, 4 or 8% by weight with respect to the weight of the uncoated tablets, were obtained. In the same manner, samples 4 to 6, each having a coating film containing hexaglycerin distearic acid ester, tetraglycerin tristearic acid ester (manufactured by Sakamoto Yakuhin Kogyo Co., Ltd.) or decaglycerin monolauric acid ester (manufactured by Riken Vitamin Co., Ltd.), whose thickness was equivalent to 2% by weight, were obtained. These samples 1 to 6 were subjected to an evaluation of the external appearance and water resistance.

### [Comparative Example 1]

A non-added sample 7 was prepared by the same method as in Example 1, except that a glycerin fatty acid ester was not contained. In addition, samples 8 and 9, each containing 100% by weight of glycerin (manufactured by Wako Pure Chemical Industries, Ltd.) with respect to the weight of chitosan, and having a coating film whose thickness was such that the weight of the coating film was equivalent to 4 or 8% by weight with respect to the weight of the uncoated tablets, were obtained. Similarly, a sample 10 containing 100% by weight of propylene glycol (manufactured by Wako Pure Chemical Industries, Ltd.), and samples 11 and 12 containing 100% by weight and 200% by weight, respectively, of sucrose monostearic acid ester (manufactured by Mitsubishi-Kagaku Foods Corp.), were obtained. These samples 7 to 12 were subjected to an evaluation of the external appearance and water resistance in the same manner as in Example 1.
The results for the evaluation of the external appearance and water resistance of the respective samples of Example 1 and Comparative Example 1 are presented in Table 1.

**[Table 1]**

| | Sample No. | Additive | | Thickness of coating film, wt% | Evaluation results | |
|---|---|---|---|---|---|---|
| | | Type | wt% | | External appearance | Water resistance |
| Example 1 | Sample 1 | Decaglycerin monopalmitic acid ester | 100 | 2 | 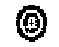 | ○ |
| | Sample 2 | | | 4 | 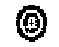 | 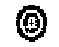 |
| | Sample 3 | | | 8 | 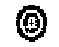 | 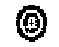 |
| | Sample 4 | Hexaglycerin distearic acid ester | 100 | 2 | 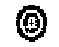 | 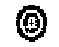 |
| | Sample 5 | Tetraglycerin tristearic acid ester | 100 | 2 | 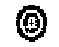 | 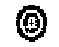 |
| | Sample 6 | Decaglycerin monolauric acid ester | 100 | 2 | 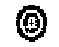 | ○ |
| Comparative Example 1 | Sample 7 | - | 0 | 8 | ⊚ | × |
| | Sample 8 | Glycerin | 100 | 4 | ⊚ | × |
| | Sample 9 | | | 8 | ⊚ | △ |
| | Sample 10 | Propylene glycol | 100 | 8 | × | × |
| | Sample 11 | Sucrose | 100 | 8 | ○ | × |
| | Sample 12 | monostearic acid ester | 200 | 8 | △ | × |

As shown in Table 1, the non-added sample 7 had been coated on the surface thereof with a chitosan coating film through a process of film coating and exhibited satisfactory external appearance, but because a large amount of organic acid remained in the coating film, the water resistance was not sufficient. Similarly, the sample 8 which had been added with glycerin, exhibited satisfactory external appearance, but water resistance was insufficient. The sample 9 whose coating film was increased the thickness to 8% by weight, also could not obtain satisfactory results. The sample 10 which had been added with propylene glycol, was insufficient in film formation, and had a problem in the external appearance. The samples 11 and 12 which had been added with sucrose monostearic acid ester, had a problem in water resistance, and it is feared that if the amount of addition is increased, the surface of the chitosan coating film would be roughened, and thus the value as commercial product may be damaged. As described above, although no additive is added, or additives such as glycerin, propylene glycol and sucrose fatty acid ester are used, these are not suitable for obtaining chitosan coating films having satisfactory water resistance (Comparative Example 1). On the contrary, the samples 1 to 6 (Example 1) which contained glycerin fatty acid esters all had no problem in the external appearance, and even though the thickness of the chitosan coating film was 2% by weight, which was thin compared to Comparative Example 1, the samples exhibited sufficient water resistance.

### Example 2

A chitosan having a degree of deacetylation of 90% by mole (manufactured by Kyowa Tecnos Co., Ltd.) was dissolved in water to a concentration or 2% by weight, after adding acetic acid so that the amount reached 2.0 molar equivalents based on the amino group of the chitosan. Then, decaglycerin monopalmitic acid ester (manufactured by Mitsubishi-Kagaku Foods Corp.) was added in an amount of 5, 10, 100 or 200% by weight with respect to the weight of chitosan, and each mixture was sufficiently stirred to obtain a chitosan solution. This solution was film coated on No. 0 gelatin hard capsules filled with starch, by using a film coating apparatus, Hicoater HC-LABO (manufactured by Freund Corporation), and thus samples 13 to 16, each having a coating film whose thickness was such that the weight of the coating film was equivalent to 2% by weight with respect to the weight of the same capsules, were obtained. In the same manner, samples 17 and 18 containing 100 and 200% by weight of tetraglycerin monostearic acid ester (manufactured by Sakamoto Yakuhin Kogyo Co., Ltd.), respectively, were obtained. These samples 13 to 18 were subjected to an evaluation of the external appearance and water resistance.

### [Comparative Example 2]

Samples 19 and 20 were prepared by the same method as in Example 2, using a chitosan solution added with 300% by weight of decaglycerin monopalmitic acid ester (manufactured by Mitsubishi-Kagaku Foods Corp.). In addition, a sample 21 which utilized tetraglycerin monostearic acid ester was obtained. These samples 19 to 21 were subjected to an evaluation of the external appearance and water resistance, in the same manner as in Example 2.
The results for the evaluation of the external appearance and water resistance of the respective samples of Example 2 and Comparative Example 2 are presented in Table 2.

**[Table 2]**

| | Sample No. | Additive | | Thickness of coating film, wt% | Evaluation results | |
|---|---|---|---|---|---|---|
| | | Type | wt% | | External appearance | Water resistance |
| Example 2 | Sample 13 | Decaglycerin monopalmitic acid ester | 5 | 2 | 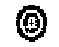 | ○ |
| | Sample 14 | | 10 | 2 | 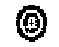 | 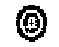 |
| | Sample 15 | | 100 | 2 | 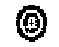 | 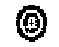 |
| | Sample 16 | | 200 | 2 | 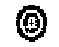 | ○ |
| | Sample 17 | Tetraglycerin monostearic acid ester | 100 | 2 | 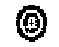 | 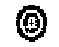 |
| | Sample 18 | | 200 | 2 | 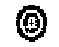 | ○ |
| Comparative Example 2 | Sample 19 | Decaglycerin monopalmitic acid ester | 300 | 2 | 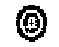 | × |
| | Sample 20 | | 300 | 8 | △ | × |
| | Sample 21 | Tetraglycerin monostearic acid ester | 250 | 8 | ○ | × |

As shown in Table 2, in the samples 19 to 21 having an amount of addition of glycerin fatty acid ester of 250% by weight or more, the proportion occupied by chitosan, which was the coating film component, was decreased, and thus the chitosan coating film could not obtain sufficient strength. Also, the external appearance was roughened because peeling occurred during the coating, or the like, and therefore, the value as commercial product was significantly damaged. Since the formation of the chitosan coating film was not sufficient, water resistance was also not sufficient (Comparative Example 2). On the contrary, the sample 13 which contained 5% by weight of a glycerin fatty acid ester with respect to the weight of chitosan, was recognized deformation in the evaluation of water resistance, but did not release the content. Also, in the case of the samples 16 and 18 which contained 200% by weight of a glycerin fatty acid ester, the chitosan coating film was obtained without any obstacle during the process of coating, and the resultant chitosan coating film had satisfactory external appearance, and did not release the content (Example 2). In order to obtain a chitosan coating film having a reduced amount of residual organic acid in the coating film and satisfactory water resistance through the process of coating, addition of a glycerin fatty acid ester is effective, and the amount of addition may desirably be in the range of 5% by weight or more and 200% by weight or less with respect to the weight of chitosan.

### Example 3

A chitosan having a degree of deacetylation of 95% by mole (manufactured by Koyo Chemical Company, Ltd.) was dissolved in water to a concentration of 2% by weight, after adding acetic acid so that the amount reached 0.8 molar equivalents based on the amino group of the chitosan. Then, hexaglycerin tristearic acid ester (manufactured by Sakamoto Yakuhin Kogyo Co., Ltd.) and propylene glycol were added to concentrations of 20% each by weight and concentrations of 50% each by weight with respect to the weight of chitosan, and the resultants were sufficiently stirred to obtain chitosan solutions. Each of the solutions was film coated on uncoated tablets having a diameter of 8 mm by using a film coating apparatus, Hicoater HC-LABO (manufactured by Freund Corporation), and thus samples 22 and 23, each having a coating film whose thickness was such that the weight of the coating film was equivalent to 2% by weight with respect to the weight of the uncoated tablets, were obtained. In the same manner, a sample 24 containing 50% by weight of decaglycerin monostearic acid ester (manufactured by Riken Vitamin Co., Ltd.) and 100% by weight of glycerin, was obtained. These samples 22 to 24 were subjected to an evaluation of the external appearance and water resistance, and the calculation of the amount of residual organic acid.

### [Comparative Example 3]

In the same manner as in Example 3, a sample 25 containing 20% by weight of hexaglycerin tristearic acid ester and 200% by weight of propylene glycol with respect to the weight of chitosan, and a sample 26 containing 50% by weight of decaglycerin monostearic acid ester and 200% by weight of glycerin, were obtained. The samples 25 and 26 were subjected to an evaluation of the external appearance and water resistance, and the calculation of the amount of residual organic acid, in the same manner as in Example 3.
The results for the evaluation of the external appearance and water resistance and the calculation of the amount of residual organic acid of the respective samples of Example 3 and Comparative Example 3 are presented in Table 3.

**[Table 3]**

| | Sample No. | Additive | | Thickness of coating film, wt% | Evaluation results | | |
|---|---|---|---|---|---|---|---|
| | | Type | wt% | | External appearance | Water resistance | Amount of residual organic acid |
| Example 3 | Sample 22 | Hexaglycerin tristearic acid ester | 20 | 2 | 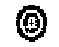 | 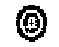 | 0. 18 |
| | | Propylene glycol | 20 | | | | |
| | Sample 23 | Hexaglycerin tristearic acid ester | 50 | 2 | 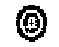 | 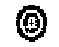 | 0. 08 |
| | | propylene glycol | 50 | | | | |
| | Sample 24 | Decaglycerin monostearic acid ester | 50 | 2 | 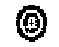 | 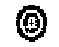 | 0. 06 |
| | | Glycerin | 100 | | | | |
| Comparative Example 3 | Sample 25 | Hexaglycerin tristearic acid ester | 20 | 2 | × | × | 0. 06 |
| | | Propylene glycol | 200 | | | | |
| | Sample 26 | Decaglycerin monostearic acid ester | 50 | 2 | 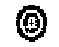 | × | 0. 06 |
| | | Glycerin | 200 | | | | |

As shown in Table 3, the samples 25 and 26 which had an amount of addition of propylene glycol or glycerin of 200% by weight, were recognized to have reduced amounts of residual organic acid. However, because a large amount of glycerin was present in the coating film, and the amount of chitosan was relatively reduced, coating films having sufficient water resistance could not be formed (Comparative Example 3). On the other hand, the samples 22 to 24 had sufficiently low values for the amount of residual organic acid, regardless of the type of glycerin fatty acid ester, and practically useful water resistance were obtained even with their relatively thin thickness of 2% by weight, (Example 3). From the above results, a chitosan coating film having sufficient water resistance can be obtained even in the case of combining a glycerin fatty acid ester with a polyhydric alcohol, in this case, it is desirable that the amount of addition of the polyhydric alcohol be in the range of 20% by weight to 100% by weight with respect to the weight of chitosan.

### Example 4

A chitosan having a degree of deacetylation of 81 % by mole (manufactured by Kyowa Tecnos Co., Ltd.) was dissolved in water to a concentration of 3% by weight, after adding acetic acid so that the amount reached 1.2 molar equivalents based on the amino group of the chitosan. Then, decaglycerin monopalmitic acid ester (manufactured by Mitsubishi-Kagaku Foods Corp.) was added to a concentration of 100% by weight with respect to the weight of chitosan, and the resultant was sufficiently stirred to obtain a chitosan solution. This solution was film coated on uncoated tablets having a diameter of 8 mm by using a film coating apparatus, Hicoater HC-LABO (manufactured by Freund Corporation), and thus a sample 27 having a coating film whose thickness was such that the weigh of the coating film was equivalent to 2, 4% by weight with respect to the weight of the uncoated tablets, was obtained. Furthermore, the coating solution of the sample 27 was stirred with a Homodisper (manufactured by Primix Corp.) to forcibly incorporate foams, and thus solutions having a foaming ratio of 1.01-food, 2.1-fold and 4.0-fold, respectively, were prepared. The resulting solutions were film coated to obtain samples 28, 29 and 30, respectively. In the same manner, a sample 31 containing 100% by weight of decaglycerin monostearic acid ester (manufactured by Riken Vitamin Co., Ltd.) and 50% by weight of glycerin was obtained, and the same solution was processed to have a foaming ratio of 1.8-food and subjected to coating, to obtain a sample 32. These samples 27 to 32 were subjected to an evaluation of the external appearance and water resistance, and the calculation of the amount of residual organic acid.

### [Comparative Example 4]

In the same manner as in Example 4, solutions added with 100% by weight of decaglycerin monopalmitic acid ester, or 100% by weight of decaglycerin monopalmitic acid ester and 50% by weight of glycerin were prepared. First, uncoated tablets were immersed in each of these solutions to coat the tablets with the chitosan solution, and then under the same conditions as in the process of film coating, except that the tablets were not subjected to introduction into a coating apparatus and spraying of the chitosan solution, only heating and drying of the tablets was carried out. After the tablet surfaces were sufficiently dried, the tablets were taken out, and the same operation was repeated until the thickness of the chitosan coating film reached the desired value of 2, 4% by weight. After the formation of chitosan coating film according to this immersion method had been completed, only those having excellent external appearance were selected by visual inspection, and samples 33 to 35 were obtained. These samples were subjected to an evaluation of the external appearance and water resistance and the calculation of the amount of residual organic acid, in the same manner as in Example 4.
The results for the evaluation of the external appearance and water resistance and the calculation of the amount of residual organic acid of the respective samples of Example 4 and Comparative Example 4 are presented in Table 4.

**[Table 4]**

| | Sample No. | Additive | | Foaming ratio, -folds | Method of coating film formation | Thickness of coating film, wt% | Evaluation results | | |
|---|---|---|---|---|---|---|---|---|---|
| | | Type | wt% | | | | External appearance | Water resistance | Amount of residual organic acid |
| Example 4 | Sample 27 | Decaglycerin monopalmitic acid ester | 100 | 1.0 | Film coating method | 2 | 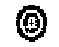 | 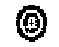 | 0.10 |
| | Sample 28 | ditto | ditto | 1.01 | | 2 | 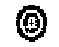 | 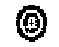 | 0.08 |
| | Sample 29 | ditto | ditto | 2.1 | | 2 | 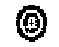 | 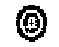 | 0.04 |
| | Sample 30 | ditto | ditto | 4.0 | | 2 | 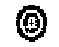 | 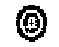 | 0.02 |
| | Sample 31 | Decaglycerin monostearic acid ester | 100 | 1.0 | | 4 | 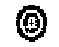 | 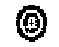 | 0.06 |
| | | Glycerin | 50 | | | | | | |
| | Sample 32 | ditto | ditto | 1.8 | | 4 | 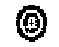 | 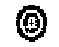 | 0.03 |
| Comparative Example 4 | Sample 33 | Decaglycerin monopalmitic acid ester | 100 | 1.0 | Immersion method | 2 | 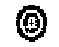 | × | 0.62 |
| | Sample 34 | ditto | ditto | 2.1 | | 2 | × | × | 0.52 |
| | Sample 35 | Decaglycerin monopalmitic acid ester | 100 | 1.0 | | 4 | 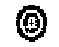 | × | 0.54 |
| | | Glycerin | 50 | | | | | | |

As shown in Table 4, the samples 33 to 35 obtained by the immersion method did not exhibit sufficient water resistance because they all had large values of the amount of residual organic acid. It is considered that in the immersion method, volatilization of the organic acid was insufficient because the surface area during drying was small (Comparative Example 4). On the contrary, the samples 27 to 32 prepared by the process of film coating had significantly reduced amounts of residual organic acid, and satisfactory water resistance were obtained. Furthermore, the samples 28, 29, 30 and 32 which contained foams such that the foaming ratio was in the range of larger than 1.0-fold to 4.0-fold or less had further reduced amounts of organic acid remaining in the chitosan coating film, as compared to samples which had the same composition but did not contain foams. For the volatilization of organic acid, the process of film coating which is capable of increasing the surface area of the chitosan solution during drying was effective, and by incorporating foams, the surface area could be increased, and the amount of residual organic acid could be further reduced (Example 4). On the other hand, an attempt was made to incorporate more foams into the sample 30 having a foaming ratio of 4.0-fold by further stirring forcibly, but it was difficult to have a foaming ratio greater than 4.0-fold. As such, it can be seen that it is desirable for a chitosan coating solution to contain foams at a foaming ratio of greater than 1.0-food and 4.0-fold or less, and the chitosan coating film obtainable by the process of film coating has a reduced amount of residual organic acid and exhibits sufficient water resistance. Furthermore, the method to obtain a chitosan coating film having a low amount of residual organic acid and satisfactory water resistance, is necessarily the process of film coating.

### Example 5

A chitosan having a degree of deacetylation of 81% by mole (manufactured by Katakura Chikkarin Co., Ltd.) was dissolved in water to a concentration of 3% by weight after adding acetic acid so that the amount reached 1.2 molar equivalents based on the amino group of the chitosan. Then, decaglycerin monopalmitic acid ester (manufactured by Mitsubishi-Kagaku Foods Corp.) was added in an amount of 100% by weight with respect to the weight of chitosan, to obtain a chitosan solution. This solution was film coated on uncoated tablets having a diameter of 8 mm using a film coating apparatus, Hicoater HCT-60N (manufactured by Freund Corporation), to obtain a thickness equivalent to 2% by weight with respect to the weight of the uncoated tablets. Subsequently, aqueous shellac (manufactured by Freund Corporation.), which is an enteric coating base, was film coated to a thickness equivalent to 4% by weight with respect to the weight of the uncoated tablets, to thus obtain a preparation sample 36, which would serve as a large intestine drug delivery system preparation. A chitosan solution which was further added with 50% by weight of glycerin with respect to the weight of chitosan and further added with foams (foaming ratio 4.0-fold), and subsequently, a preparation sample 37 coated with aqueous shellac, and a preparation sample 38 coated with hypromellose phthalic acid ester (manufactured by Shin-Etsu Chemical Co., Ltd.) instead of aqueous shellac, were obtained. Furthermore, a preparation sample 39 coated with a chitosan solution (foaming ratio 1.8-fold) which contained 100% by weight of decaglycerin tristearic acid ester and 50% by weight of glycerin with respect to the weight of chitosan, and with hypromellose phthalic acid ester was obtained. These samples 36 to 39 were subjected to an evaluation of the external appearance and the in vitro large intestine DDS test.

### [Comparative Example 5]

In the same manner as in Example 5, a preparation sample 40 coated with a non-added chitosan solution and then with aqueous shellac, was obtained. In the same manner, there were obtained a preparation sample 41 coated with a chitosan solution containing 50% by weight of glycerin with respect to the weight of chitosan, and with aqueous shellac, and a preparation sample 42 coated with a chitosan solution containing 100% by weight of sucrose monostearic acid ester with respect to the weight of chitosan, and with hypromellose phthalic acid ester. These samples 40 to 42 were subjected to an evaluation of the external appearance and the in vitro large intestine DDS test.
The results for the external appearance and the in vitro large intestine DDS test for the respective samples of Example 5 and Comparative Example 5 are presented in Table 5.

**[Table 5]**

| | Sample No. | Additive | | Foaming ratio -fold | Thickness of coating film, wt% | Enteric coat, wt% | Evaluation results | | | |
|---|---|---|---|---|---|---|---|---|---|---|
| | | Type | wt% | | | | External appearance | Large intestine DDS | | |
| | | | | | | | | Pharma- copoeia 1 | Pharma- copoeia 2 | Acetic acid |
| Example 5 | Preparation sample 36 | Decaglycerin monopalmitic acid ester | 100 | 1.0 | 2 | Aqueous shellac 4 | 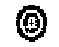 | ○ | ○ | ○ |
| | Preparation sample 37 | Decaglycerin monopalmitic acid ester | 100 | 4.0 | 2 | Aqueous shellac 4 | 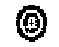 | ○ | ○ | ○ |
| | | Glycerin | 50 | | | | | | | |
| | Preparation sample 38 | ditto | ditto | 4.0 | 2 | Hypromellose phthalic acid ester 4 | 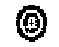 | ○ | ○ | ○ |
| | Preparation sample 39 | Decaglycerin tristearic acid ester | 100 | 1.8 | 2 | Hypromellose phthalic acid ester 4 | 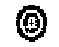 | ○ | ○ | ○ |
| | | Glycerin | 50 | | | | | | | |
| Comparative Example 5 | Preparation sample 40 | - | 0 | 1.0 | 2 | Aqueous shellac 4 | 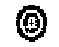 | ○ | × | - |
| | Preparation sample 41 | Glycerin | 50 | 1.0 | 2 | Aqueous shellac 4 | 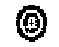 | ○ | × | - |
| | Preparation sample 42 | Sucrose monostearic acid ester | 100 | 1.2 | 2 | Hypromellose phthalic acid ester 4 | 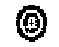 | ○ | × | - |

From the results in Table 5, none of the preparation samples had particular problems in the resistance to Japanese Pharmacopoeia first fluid, but the preparation samples 40 to 42 did not obtain the resistance to Japanese Pharmacopoeia second fluid, and released the contents. Thus, the function of the preparation samples as large intestine drug delivery system preparations cannot be sufficient, and lacks practicality (Comparative Example 5). On the other hand, the preparation samples 36 to 39 which contained a glycerin fatty acid ester, exhibited sufficient resistance to Japanese Pharmacopoeia second fluid, and had no problem in the strength of the chitosan coating film, subsequently releasing the contents rapidly in an acetate buffer solution (Example 5). Therefore, a combination of the chitosan coating film of the present invention and an enteric coating film exhibits excellent functions as a large intestine drug delivery system preparation.

## Claims

1. A chitosan coating solution comprising an aqueous solution of chitosan dissolved using a volatile organic acid, and 5% by weight to 200% by weight of a glycerin fatty acid ester with respect to the weight of chitosan.

2. The coating solution according to claim 1 further comprising 20% by weight to 100% by weight of a polyhydric alcohol with respect to the weight of chitosan.

3. The coating solution according to claim 1 or 2, comprising foams such that the expansion ratio is in the range of greater than 1.0-fold to 4.0-fold or less.

4. A chitosan coating film, formed by film-coating the solution according to any one of claims 1 to 3 on a surface of a substrate.

5. A chitosan coated preparation, prepared by forming a chitosan coating film formed by film-coating the solution according to any one of claims 1 to 3 on a surface of a substrate.

6. The chitosan coated preparation according to claim 5, wherein the substrate is a food product or a pharmaceutical product.

7. A large intestine drug delivery system preparation, comprising the chitosan coated preparation according to claim 6, and an enteric coating film coated on the outer layer of the chitosan coated preparation.
